# EUROPÄISCHE PATENTANMELDUNG

(11) **EP 3 816 123 A1**
(43) Veröffentlichungstag der Anmeldung: **05.05.2021**
(21) Anmeldenummer: 20165081.9
(22) Anmeldetag: 24.03.2020
(51) Int. Cl.: C03C 17/00, C03C 17/42, G01N 33/00, G01N 21/29, G01N 21/78

(54) **GASOCHROMES GLAS, VERFAHREN ZUR HERSTELLUNG DESSELBEN UND VORRICHTUNG ZUR DETEKTION EINES ZIELGASES**

(71) Anmelder: FRAUNHOFER-GESELLSCHAFT zur Förderung der angewandten Forschung e.V., 80686 München (DE)
(72) Erfinder: BAUERSFELD, Marie-Luise, 79110 Freiburg (DE); PANNEK, Carolin, 79110 Freiburg (DE); DOLD, Martin, 79110 Freiburg (DE)
(74) Vertreter: Lemcke, Brommer & Partner Patentanwälte Partnerschaft mbB

(57) **Zusammenfassung**

Vorgeschlagen wird ein gasochrom beschichtetes Glas (1) für einen Gasdetektor (11), mit einem Träger (2) mit einer Trägeroberfläche (3); und einem gasochromen Stoff (7); welches sich dadurch auszeichnet, dass eine poröse Trägerschicht (4) aus SiO₂-Partikeln (5) an der Trägeroberfläche (3) angeordnet ist und dass der gasochrome Stoff (7) an der porösen Trägerschicht (4) angeordnet ist.

## Beschreibung

Die Erfindung betrifft ein gasochrom beschichtetes Glas für einen Gasdetektor gemäß Anspruch 1, eine Vorrichtung zur Detektion eines Zielgases gemäß Anspruch 10, ein Verfahren zur Herstellung eines gasochrom beschichteten Glases gemäß Anspruch 11 sowie die Verwendung einer Vorrichtung zur Detektion eines Zielgases gemäß Anspruch 14.

Gasochrome Stoffe oder Materialien wechselwirken und/oder reagieren mit gasförmigen Stoffen. Durch die Wechselwirkung und/oder die Reaktion mit einem gasförmigen Stoff ändert der gasochrome Stoff seine physikochemischen Eigenschaften. Durch die Reaktion des gasochromen Stoffs mit einem Zielgas erfolgt typischerweise eine Farbänderung oder ein Farbumschlag, also eine Änderung im elektromagnetischen Absorptionsspektrum des gasochromen Stoffs. Die Farbänderung kann von einer optischen Detektionseinheit in ein Messsignal umgewandelt werden. Bei der Farbänderung ändert sich das elektromagnetische Absorptionsspektrum des gasochromen Stoffs typischerweise in einem Wellenlängenbereich des sichtbaren Lichts (im UV-Vis-Bereich von 380 nm bis 780 nm), wodurch die Reaktion des gasochromen Stoffs mit dem gasförmigen Stoff durch eine resultierende Farbänderung des gasochromen Stoffs in einfacher Weise optisch detektierbar ist. Gasochrome Stoffe und Materialien sind aufgrund ihrer vorbeschriebenen Eigenschaften insbesondere dazu geeignet, in Gasdetektoren eingesetzt zu werden, typischerweise um die Anwesenheit des Zielgases, mit welchem der gasochrome Stoff wechselwirkt, zu detektieren.

Aus der CN 102759525 B sind Gasdetektoren bekannt, bei denen ein gasochromer Stoff, beispielsweise ein Anthocyanin, auf einem porösen Titandioxidfilm (TiO₂-Film) abgeschieden wird. Zur Herstellung des TiO₂-Films wird ein TiO₂-Puder mit einem sogenannten OP-Emulsifier (Alkylphenol polyoxyethylen ether, also ein organisches Polymer) und weiteren Zusatzstoffen vermischt und auf einen Träger, beispielsweise einen Siliziumchip, eine elektrisch leitende Glasplatte oder einer Teflonplatte aufgebracht.

Nachteilig an diesem Stand der Technik ist, dass Titandioxid einen hohen Brechungsindex aufweist und somit einfallendes Licht stark streut. Titandioxid kommt deshalb häufig als Weißpigment zum Einsatz, beispielsweise bei Lacken, Anstrichen bzw. Anstrichfarben oder in UV-Schutzhautcremes. Durch diese intensive Lichtstreuung werden optische Detektionsmethoden in vielfältiger Weise gestört. Insbesondere kommt es bei intensiver Lichtstreuung aufgrund von zahlreichen Störreflexionen zu großen Messungenauigkeiten. Des Weiteren ist eine optische Transmissionsmessung durch den TiO₂-Film hindurch stark beeinträchtigt oder gar nicht möglich.

Ausgehend von diesem Stand der Technik liegt der vorliegenden Erfindung die Aufgabe zugrunde, ein gasochrom beschichtetes Glas sowie eine Vorrichtung zur Detektion eines Zielgases bereitzustellen, welche kostengünstig und dennoch langzeitstabil sind. Zudem liegt der vorliegenden Erfindung die Aufgabe zugrunde, ein Verfahren zur Herstellung eines gasochrom beschichteten Glases bereitzustellen, welches kostengünstig und dennoch effizient ist.

Diese Aufgabe wird erfindungsgemäß gelöst durch ein gasochrom beschichtetes Glas mit den Merkmalen des Anspruchs 1, durch eine Vorrichtung zur Detektion eines Zielgases mit den Merkmalen des Anspruchs 11, durch ein Verfahren zur Herstellung eines gasochrom beschichteten Glases mit den Merkmalen des Anspruchs 12 und die Verwendung einer Vorrichtung zur Detektion eines Zielgases mit den Merkmalen des Anspruchs 15. Vorteilhafte Ausführungsformen sind Gegenstand von Unteransprüchen.

Ein erfindungsgemäßes, gasochrom beschichtetes Glas für einen Gasdetektor weist einen Träger mit einer Trägeroberfläche, einen gasochromen Stoff und eine poröse Trägerschicht auf, welche Siliziumdioxid-Partikel (SiO₂-Partikel) aufweist. Die poröse Trägerschicht ist an der Trägeroberfläche angeordnet. Erfindungsgemäß ist der gasochrome Stoff an den SiO₂-Partikeln der porösen Trägerschicht angeordnet.

Die SiO₂-Partikel machen vorzugsweise einen Massenanteil der porösen Trägerschicht von größer gleich 30% bis kleiner gleich 100% aus, besonders vorzugsweise von größer gleich 80% bis kleiner gleich 100% aus. Höchst vorzugsweise besteht die poröse Schicht aus SiO₂-Partikeln.

Durch das Vorsehen der porösen Trägerschicht wird in vorteilhafter Weise die mit dem Zielgas in Verbindung tretende äußere Oberfläche vergrößert, sodass eine höhere Detektionseffizienz der gasochromen Moleküle erreicht wird. Ein weiterer Vorteil besteht darin, dass die Trägerschicht chemisch besonders inert ist, das bedeutet, dass die Trägerschicht insbesondere nicht mit gasochromen Stoffen reagiert. Zudem ist die Trägerschicht chemisch unbedenklich. Die Trägerschicht ist vorteilhafterweise stabil gegenüber Umwelteinflüssen wie UV-Strahlung, schwankender Luftfeuchtigkeit und Temperaturen von über 80 °C. Somit ist die Trägerschicht besonders langzeitstabil.

Zielgase im Sinne der vorliegenden Beschreibung sind insbesondere Treibhausgase, Umweltgifte, Rauch, flüchtige organische Verbindungen (volatile organic compounds - VOC) und/oder Aromastoffe. Vorzugsweise ist das Zielgas Kohlenstoffmonoxid (CO), Kohlenstoffdioxid (CO₂), Stickstoffmonoxid (NO), ein Stickoxid (NOₓ), insbesondere Stickstoffdioxid (NO₂), Dimethylsulfid (SMe₂), Schwefeldioxid (SO₂), Schwefelwasserstoff (H₂S), und/oder Ammoniak (NH₃).

Es liegt im Rahmen der Erfindung, dass das gasochrom beschichtete Glas ein Polymer aufweist. Der Begriff Polymer umfasst im Sinne dieser Beschreibung insbesondere organische Polymere, die aus organischen Monomeren gebildet sind und/oder anorganische Polymere, die aus anorganischen Monomeren gebildet sind. Nachteilig ist hierbei jedoch, dass in Polymere eingebettete gasochrome Stoffe schlechter, insbesondere langsamer oder gar nicht mit dem Zielgas in Kontakt treten. Das Gleiche gilt für gasochrome Stoffe, die an schlecht zugänglichen inneren Oberflächen eines Polymernetzwerkes, beispielsweise eines Siliziumdioxid-Netzwerks, angeordnet sind.

Daher ist bei einer vorteilhaften Ausführungsform der gasochrome Stoff nicht in ein Polymer eingebettet. Damit ist vorzugsweise umfasst, dass der gasochrome Stoff nicht in einen Polymerfilm oder in eine Polymermatrix eingebettet ist. Vorzugsweise wird der gasochrome Stoff von dem Polymer nicht, zumindest teilweise umhüllt und/oder umschlossen und/oder eingeschlossen. Besonders vorzugsweise ist der gasochrome Stoff nicht an einer inneren Oberfläche eines Polymernetzwerkes angeordnet, insbesondere nicht an inneren Oberflächen eines Siliziumdioxid-Netzwerks.

Somit wird bei dieser Ausführungsform die Wechselwirkung mit dem Zielgas vorteilhafter Weise durch eine verbesserte Zugänglichkeit und eine vergrößerte Oberfläche erhöht. Somit wird die Detektionseffizienz gesteigert, insbesondere erfolgt eine schnellere Detektion.

Bei einer weiteren vorteilhaften Ausführungsform ist das gasochrom beschichtete Glas frei von organischen Polymeren.

Bei Versuchen der Erfinder wurde erkannt, dass es bei gasochromen Gläsern nachteilig ist, organische Polymere einzusetzen, insbesondere als Haftvermittlersubstanzen, weil organische Polymere die Lebensdauer eines gasochromen Gasdetektors unvorteilhaft verkürzen. Organische Polymere sind anfällig für Alterungsprozesse wie beispielsweise UV-Schäden, Oxidation, Polymerkettendegradation, Verflüchtigung von Weichmachern oder dergleichen. Abhängig von dem zu detektierenden Zielgas und dem jeweils eingesetzten Polymer kann auch eine Reaktion des Polymers mit dem Zielgas stattfinden, wodurch sich zum einen das Polymer verändern, insbesondere zersetzen kann und zum anderen das Zielgas möglicherweise nicht mehr in ausreichender Konzentration zur Detektion zur Verfügung steht. Darüber hinaus können organische Polymere durch eine Eigenfärbung der Polymermatrix, insbesondere aufgrund der zuvor genannten Alterungsprozesse oder Reaktionen mit dem Zielgas die Detektion des Zielgases verschlechtern, da eine Verfälschung der Farbänderung durch die (gefärbte) Polymermatrix bewirkt wird. Viele organische Polymere sind außerdem empfindlich gegenüber Umwelteinflüssen wie beispielsweise UV-Strahlung, schwankender Luftfeuchtigkeit und besonders hohen und/oder niedrigen Temperaturen. Bei erhöhter Luftfeuchtigkeit können viele organische Polymere unvorteilhaft aufquellen. Durch die zuvor genannte Ausführungsform wird also die Langlebigkeit noch weiter erhöht und die Detektionseffizienz weiter verbessert. Bei einer vorteilhaften Ausführungsform ist der gasochrome Stoff dazu ausgebildet, durch die Einwirkung eines Zielgases eine Farbänderung aufzuweisen. Die Farbänderung führt bevorzugt zu einer geänderten Farbe des gasochromen Stoffs. Vorzugsweise ist die Trägerschicht transparent für Licht zumindest im Wellenlängenbereich der geänderten Farbe, insbesondere in einem Wellenlängenbereich einer charakteristischen Absorptionsbande der geänderten Farbe und/oder für Licht im UV-Vis-Bereich. Besonders vorteilhaft ist die Trägerschicht transparent für Licht in einem Wellenlängenbereich von größer gleich 350 nm bis kleiner gleich 800 nm.

Somit ist in vorteilhafter Weise die Farbänderung des gasochromen Stoffs durch eine Transmissionsmessung durch die Trägerschicht hindurch detektierbar.

Bei einer weiteren vorteilhaften Ausführungsform umfasst die Trägerschicht zumindest einen ersten Teilbereich und einen zweiten Teilbereich. Der gasochrome Stoff umfasst bei dieser Ausführungsform vorzugsweise einen ersten gasochromen Stoff und einen zweiten gasochromen Stoff, wobei der erste gasochrome Stoff an dem ersten Teilbereich und der zweite gasochrome Stoff an dem zweiten Teilbereich angeordnet ist. Der erste Teilbereich und der zweite Teilbereich sind vorzugsweise räumlich voneinander getrennt.

Dabei ist vorzugsweise der erste gasochrome Stoff zur Detektion eines ersten Zielgases und der zweite gasochrome Stoff zur Detektion eines zweiten Zielgases ausgebildet, welches unterschiedlich zu dem ersten Zielgas ist. Vorteilhafterweise werden durch zwei unterschiedliche gasochrome Stoffe somit unterschiedliche Zielgase detektiert. Durch die räumlich voneinander getrennte Anordnung der Teilbereiche wird die parallele Detektion von mehreren Zielgasen vereinfacht.

Bei einer vorteilhaften Ausführungsform ist in dem ersten Teilbereich und in dem zweiten Teilbereich der gleiche gasochrome Stoff in unterschiedlichen Konzentrationen angeordnet. Vorzugsweise ist die Konzentration des gasochromen Stoffs in dem zweiten Teilbereich größer als in dem ersten Teilbereich. Dadurch wird vorteilhafterweise eine konzentrationsabhängige Detektion des Zielgases ermöglicht.

Bei einer bevorzugten Ausführungsform umfasst die poröse Trägerschicht zumindest eine erste Lage von SiO₂-Partikeln und zumindest eine zweite Lage von SiO₂-Partikeln. Dabei ist die erste Lage zwischen dem Träger und der zweiten Lage angeordnet. Der gasochrome Stoff ist an den SiO₂-Partikeln der ersten Lage angeordnet, vorzugsweise an einer äußeren porösen Oberfläche der SiO₂-Partikel der ersten Lage. Somit befindet sich der gasochrome Stoff vorzugsweise zwischen dem Träger und der zweiten Lage an der Oberfläche der SiO₂-Partikel der ersten Lage. Die zweite Lage von SiO₂-Partikeln weist bevorzugt keinen gasochromen Stoff auf. Diese Ausführungsform hat den Vorteil, dass der gasochrome Stoff gegenüber mechanischem Abrieb oder sonstiger Erosion besser geschützt ist. Somit wird die Langlebigkeit weiter verbessert.

Bei einer bevorzugten Ausführungsform umfasst die poröse Trägerschicht SiO₂-Partikel, vorzugsweise miteinander verbundene SiO₂-Partikel. Dadurch wird die Trägerschicht stabiler und somit wird die Langlebigkeit erhöht. SiO₂-Partikel werden besonders gut von dem Zielgas umströmt. Somit wird die Detektion vorteilhafterweise verbessert.

Bei einer weiteren Ausführungsform ist die poröse Trägerschicht aus einzelnen voneinander separierten und bevorzugt im Abstand angeordneten SiO₂-Partikeln gebildet. Dadurch werden die SiO₂-Partikel besonders gut von dem Zielgas umströmt, wodurch die Detektionseffizienz verbessert wird.

Bei einer besonders bevorzugten Ausführungsform ist der Träger optisch transparent, vorzugsweise für Licht im Wellenlängenbereich der geänderten Farbe, besonders vorzugsweise im Wellenlängenbereich einer charakteristischen Absorptionsbande der geänderten Farbe und/oder im UV-Vis-Wellenlängenbereich. Höchst vorzugsweise ist der Träger aus Glas ausgebildet. Dadurch wird die Detektion weiter verbessert und eine Transmissionsmessung durch den Träger hindurch vereinfacht.

Bei einer weiteren vorteilhaften Ausführungsform sind die SiO₂-Partikel mit der Trägeroberfläche verbunden, vorzugsweise stoffschlüssig verbunden. Bei einer bevorzugten Ausführungsform ist der gasochrome Stoff mit den SiO₂-Partikeln verbunden, vorzugsweise stoffschlüssig. Durch die beiden zuvor genannten Ausführungsformen wird die Langlebigkeit weiter erhöht.

Bei einer weiteren vorteilhaften Ausführungsform sind die SiO₂-Partikel als SiO₂-Nanopartikel ausgebildet, vorzugsweise in einem Größenbereich von kleiner gleich 5 nm bis größer gleich 200 nm, insbesondere bevorzugt kleiner gleich 10 nm bis größer gleich 150 nm, höchst vorzugsweise kleiner gleich 80 nm bis größer gleich 110 nm, weiter höchst vorzugsweise 95 nm. SiO₂-Nanopartikel in diesen Größenbereichen werden besonders gut von dem Zielgas umströmt, wodurch sich die Detektionseffizienz verbessert. SiO₂-Nanopartikel mit einer Größe von 95 nm sind zusätzlich besonders kosteneffizient und einfach in der Herstellung.

Die SiO₂-Nanopartikel werden bevorzugt in einem Verfahren gemäß der EP 0 835 849 B1 hergestellt. Durch SiO₂-Nanopartikel wird die äußere Oberfläche vorteilhafterweise vergrößert, wodurch die Detektionseffizienz verbessert wird.

Bei einer besonders bevorzugten Ausführungsform sind die SiO₂-Partikel als Agglomerate von SiO₂-Nanopartikeln ausgebildet, vorzugsweise als Suprapartikel.

Solche Suprapartikel sind aus der Veröffentlichung von Wintzheimer et al. "Indicator Supraparticles for Smart Gasochromic Sensor Surfaces Reacting Ultrafast and Highly Sensitive", erschienen in "Particle and Particle Sensor Systems", im Jahr 2019, DOI 10.1002/ppsc.201900254, bekannt. Die Herstellung der Suprapartikel erfolgt vorzugsweise über ein Sol-Gel-Verfahren.

Die Suprapartikel weisen vorzugsweise einen Durchmesser von größer gleich 4 µm bis kleiner gleich 100 µm auf, besonders vorzugsweise größer gleich 5 µm bis kleiner gleich 80 µm, höchst vorzugsweise größer gleich 30 µm bis kleiner gleich 70 µm.

Besonders bevorzugt weisen die Suprapartikel eine spezifische Oberfläche von größer gleich 40 m²g⁻¹ bis kleiner gleich 180 m²g⁻¹ auf, vorzugsweise größer gleich 45 m²g⁻¹ bis kleiner gleich 100 m²g⁻¹.

Suprapartikel mit einem Durchmesser und/oder mit einer spezifischen Oberfläche in dem/den zuvor genannten Größenbereich/Größenbereichen werden besonders gut von dem Zielgas umströmt, wodurch sich die Detektionseffizienz weiter verbessert.

Vorteilhafterweise weist die Trägerschicht aus Suprapartikeln im Vergleich zu einzelnen nicht agglomerierten SiO₂-Nanopartikeln eine besonders große Oberfläche und ein großes Oberfläche-zu-Volumen-Verhältnis auf, wodurch sich der gasochrome Stoff besser auf der Oberfläche verteilen lässt und schnelle Reaktionszeiten mit dem Zielgas resultieren. Darüber hinaus ist der gasochrome Stoff auf der gerundet strukturierten Oberfläche der Suprapartikel für das Zielgas besonders gut zugänglich, das heißt, das Zielgas kann die Suprapartikel besonders gut umströmen und den gasochromen Stoff gut erreichen, wodurch die Detektionseffizienz weiter verbessert wird. Zudem lässt sich die Oberfläche der Suprapartikel besonders einfach und effektiv mit dem gasochromen Stoff beschichten, wodurch Kosten bei der Produktion gespart werden.

Bei einer bevorzugten Ausführungsform weist die Trägeroberfläche keine Strukturierung oder Profilierung auf, insbesondere ist die Trägeroberfläche bevorzugt eben ausgebildet. Dadurch wird die Anordnung der Trägerschicht an der Trägeroberfläche vereinfacht und reproduzierbarer.

Bei einer bevorzugten Ausführungsform ist die Trägeroberfläche zumindest teilweise mit der Trägerschicht beschichtet. Dabei sind die SiO₂-Partikel der Trägerschicht entweder dicht gepackt oder lose gepackt. Bei einer dichten Packung weist die Trägeroberfläche insbesondere eine hohe Besetzungszahl der SiO₂-Partikel auf, wobei sich vorzugsweise einzelne SiO₂-Partikel gegenseitig berühren. Bei einer losen Packung weist die Trägeroberfläche insbesondere eine niedrige Besetzungszahl der SiO₂-Partikel auf, wobei sich vorzugsweise einzelne SiO₂-Partikel nicht berühren. Vorzugsweise weist die Trägeroberfläche in dem ersten Teilbereich eine hohe Besetzungszahl auf und in einem zweiten Teilbereich eine niedrige Besetzungszahl auf. Dadurch lässt sich die Detektion vorteilhafterweise flexibel an bestimmte Anwendungsbereiche anpassen.

Die Trägeroberfläche, deren Besetzungszahl, die Trägerschicht und die SiO₂-Partikel können über Mikroskopie, insbesondere über Rasterelektronenmikroskopie (REM) und Photoelektronenspektroskopie (XPS) charakterisiert werden.

Bei einer weiteren Ausführungsform ist die Trägerschicht unmittelbar an der Trägeroberfläche angeordnet, wobei vorzugsweise keine haftvermittelnde Substanz und/oder kein organisches Polymer zwischen der Trägerschicht und der Trägeroberfläche angeordnet ist. Dadurch wird die Langlebigkeit in vorteilhafter Weise erhöht.

Bei einer bevorzugten Ausführungsform ist der gasochrome Stoff unmittelbar an der porösen Trägerschicht angeordnet, vorzugsweise an einer äußeren Oberfläche der SiO₂-Partikel. Bevorzugt ist die poröse Trägerschicht mit dem gasochromen Stoff zumindest teilweise beschichtet und/oder bedeckt. Zwischen dem gasochromen Stoff und der porösen Trägerschicht ist vorzugsweise keine haftvermittelnde Substanz und/oder kein organisches Polymer angeordnet. Dadurch wird die Langlebigkeit vorteilhafterweise erhöht.

Bei einer weiteren vorteilhaften Ausführungsform ist der gasochrome Stoff nicht innerhalb der SiO₂-Partikel angeordnet. Dadurch wird in vorteilhafter Weise die Detektionseffizienz verbessert. Zudem wird eine geringere Menge des gasochromen Stoffs benötigt.

Bei einer weiteren vorteilhaften Ausführungsform ist der gasochrome Stoff nicht an einer inneren Oberfläche eines Polymernetzwerkes angeordnet, insbesondere nicht an einer inneren Oberfläche eines Siliziumdioxid-Netzwerks. Die Polymernetzwerke weisen in Abgrenzung zu der Trägerschicht keine SiO₂-Partikel auf, sondern weisen eine quervernetzte und/oder gerüstartige Struktur auf. Gasochrome Stoffe an schlecht zugänglichen inneren Oberflächen des Polymernetzwerkes, treten schlechter, insbesondere langsamer oder gar nicht mit dem Zielgas in Kontakt. Somit wird durch diese Ausführungsform die Detektionseffizienz weiter verbessert.

Bei einer bevorzugten Ausführungsform ist der gasochrome Stoff aus der Tabelle 1 gewählt. Die gasochromen Stoffe aus der linken Spalte der Tabelle 1 eignen sich insbesondere zur Detektion der entsprechenden Zielgase der rechten Spalte der Tabelle 1.

**Tabelle 1**

| Gasochromer Stoff | Zielgas |
|---|---|
| N,N,N',N'-Tetramethyl-p-phenylendiamin, Alexey et al. DOI: 10.1016/j.snb.2005.08.041 | Stickstoffdioxid (NO₂) |
| | |
| Bromphenolblau, Courbat et. al. DOI: 10.1016/j.snb.2009.08.049 | Ammoniak (NH₃) |
| | |
| Rhodiumkomplexe, wie aus der Veröffentlichung von Pannek et al. "Investigation of Gasochromic Rhodium Complexes Towards Their Reactivity to CO and Integration into an Optical Gas Sensor for Fire Gas Detection", erschienen in Sensors, 2018, 18(7), DOI: 10.3390/s18071994, bekannt | Kohlenstoffmonoxid (CO) |
| | |
| Molybdänblau, welches aus der Redoxreaktion von Ethylen mit Ammoniummolybdat entsteht, Lang et al. DOI: 10.1007/s11947-011-0694-4 | Ethylen (C₂H₄) |

Eine erfindungsgemäße Vorrichtung zur Detektion eines Zielgases ist vorzugsweise als Gasdetektor ausgebildet und umfasst ein erfindungsgemäßes gasochrom beschichtetes Glas, insbesondere eine vorteilhafte Ausführung hiervon. Darüber hinaus umfasst die erfindungsgemäße Vorrichtung einen Detektor und einen Detektionsbereich. Bei der erfindungsgemäßen Vorrichtung ist der gasochrome Stoff zumindest in dem Detektionsbereich angeordnet und der Detektor dazu ausgebildet, abhängig von einer Farbänderung des Detektionsbereichs das Zielgas zu detektieren. Die erfindungsgemäße Vorrichtung ist aufgrund der zuvor beschriebenen vorteilhaften Eigenschaften des gasochrom beschichteten Glases besonders langlebig, sensitiv und effizient.

Bei einer bevorzugten Ausführungsform ist der Detektor in Transmissionsmesskonfiguration angeordnet, vorzugsweise zum Messen von Licht, welches durch das gasochrom beschichtete Glas transmittiert. Vorteilhaft ist, dass bei dieser Ausführungsform auch Sonnenlicht oder Licht einer externen Lichtquelle zur Detektion genutzt wird. Dadurch ist der Gasdetektor besonders robust und langlebig.

Bei einer weiteren bevorzugten Ausführungsform umfasst die Vorrichtung eine Lichtquelle, welche dazu ausgebildet ist, zumindest den Detektionsbereich mit Licht zu bestrahlen. Vorzugsweise ist das gasochrom beschichtete Glas zwischen der Lichtquelle und dem Detektor in Transmissionsmesskonfiguration angeordnet. Die Lichtquelle ist besonders vorzugsweise in Reflexionsmesskonfiguration zu dem Detektor angeordnet. Somit wird eine vorteilhafte und effiziente Alternative zur Transmissionsmessung realisiert.

Bei einer vorteilhaften Ausführungsform ist die Lichtquelle eine interne Lichtquelle. Diese Ausführungsform hat den Vorteil, dass die Vorrichtung unabhängig von externen Lichtquellen und/oder dem Sonnenlicht betrieben wird, und insbesondere auch bei Nacht eingesetzt werden kann.

Bei einer weiteren Ausführungsform ist die Lichtquelle dazu ausgebildet, Licht in definierten und/oder einstellbaren Wellenlängen und/oder Wellenlängenbereichen auszustrahlen. Dadurch ist vorteilhafterweise die gezielte Bestrahlung des gasochromen Stoffs mit Licht in unterschiedlichen und/oder vorbestimmten Wellenlängen/-bereichen möglich, wodurch die Messgenauigkeit verbessert wird.

Bei einer weiteren Ausführungsform umfasst die Vorrichtung eine Schutzschicht, insbesondere eine zielgasdurchlässige Schutzschicht. Die Schutzschicht ist vorzugsweise über der Trägerschicht angeordnet. Vorteilhafterweise schützt die Schutzschicht die Trägerschicht vor Beschädigungen, insbesondere vor Abrieb.

Ein erfindungsgemäßes Verfahren zur Herstellung eines gasochrom beschichteten Glases, vorzugsweise eines gasochrom beschichteten Glases der zuvor beschriebenen Art, umfasst die Schritte:
- Bereitstellen eines Trägers mit einer Trägeroberfläche; einer Vielzahl von SiO₂-Partikeln; und eines gasochromen Stoffs;
- Anordnen der SiO₂-Partikel an der Trägeroberfläche;
- Anordnen des gasochromen Stoffs an den SiO₂-Partikeln.

Durch das erfindungsgemäße Verfahren lässt sich ein gasochrom beschichtetes Glas besonders einfach, schnell und effizient herstellen.

Bei einer bevorzugten Ausführungsform erfolgt kein Einbetten des gasochromen Stoffs in ein Polymer. Vorzugsweise werden keine organischen Polymere zur Fixierung der SiO₂-Partikel an der Trägeroberfläche oder des gasochromen Stoffs an den SiO₂-Partikeln eingesetzt. Dadurch ergeben sich die schon zuvor genannten Vorteile, insbesondere eine erhöhte Langlebigkeit des zu erzeugenden gasochrom beschichteten Glases und eine verbesserte Detektionseffizienz. Des Weiteren wird das Verfahren dadurch einfacher, schneller und kosteneffizienter.

Bei einer bevorzugten Ausführungsform erfolgt nach dem Anordnen der SiO₂-Partikel an der Trägeroberfläche ein Erhitzen, vorzugsweise ein Tempern, besonders vorzugsweise bei einer Temperatur im Bereich von größer gleich 300 °C bis kleiner gleich 700 °C, höchst vorzugsweise bei 500 °C. Bevorzugt wird der gasochrome Stoff nachfolgend an den SiO₂-Partikeln angeordnet, also nach dem Erhitzen. Dadurch lässt sich, wie zuvor beim Tempern beschrieben, eine höhere Qualität des zu erzeugenden gasochrom beschichteten Glases erzielen, insbesondere da die SiO₂-Partikel durch das Erhitzen stabiler gegenüber einem Abrieb von der Trägeroberfläche sind.

Bei einer bevorzugten Ausführungsform wird die poröse Trägerschicht auf die Trägeroberfläche aufgesintert und/oder aufgeschmolzen. Beim Sintern erfolgt ein Tempern unterhalb des Schmelzpunktes der SiO₂-Partikel und/oder des Trägers, wohingegen beim Aufschmelzen ein Tempern am Schmelzpunkt der SiO₂-Partikel und/oder des Trägers erfolgt.

Die auf der Trägeroberfläche aufgesinterte und/oder aufgeschmolzene Trägerschicht ist vorteilhafterweise besonders stabil gegenüber mechanischen Belastungen wie beispielsweise Abrieb und damit langlebig.

Bei einer weiteren Ausführungsform erfolgt das Anordnen des gasochromen Stoffs nach dem Erhitzen. Dadurch werden temperatursensitive gasochrome Stoffe in vorteilhafter Weise geschont.

Bei einer vorteilhaften Ausführungsform wird der gasochrome Stoff zunächst in einem Lösungsmittel gelöst und/oder suspendiert, wodurch eine Lösung und/oder eine Suspension erhalten wird. Nachfolgend wird der gasochrome Stoff aus der Lösung und/oder aus der Suspension auf die Trägerschicht aufgebracht. Dadurch wird eine besonders hohe Benetzung der porösen Oberfläche mit dem gasochromen Stoff erreicht. Zudem erfolgt eine effektive und bessere Verteilung des gasochromen Stoffs an der äußeren porösen Oberfläche der SiO₂-Partikel, wodurch eine gute Zugänglichkeit des gasochromen Stoffs für das Zielgas erreicht wird und somit die Detektionssensitivität verbessert wird.

Bei einer bevorzugten Ausführungsform umfasst das Verfahren den Schritt
- Trocknen, vorzugsweise Trocknen bei einer Temperatur in einem Bereich von größer gleich 40 °C bis kleiner gleich 100 °C, besonders vorzugsweise bei 60 °C, insbesondere bei einem Druck von 10 mbar.

Dadurch wird vorteilhafterweise etwaig vorhandenes (Rest-) Lösungsmittel entfernt.

Bei einer weiteren vorteilhaften Ausführungsform wird der gasochrome Stoff an einer porösen Oberfläche der SiO₂-Partikel abgeschieden, vorzugsweise durch Gasphasenabscheidung und/oder durch Aufsublimieren des gasochromen Stoffs auf die poröse Oberfläche. Dadurch wird das Aufbringen des gasochromen Stoffs vorteilhafterweise vereinfacht. Zudem wird der gasochrome Stoff an besonders gut zugänglichen Bereichen der äußeren porösen Oberfläche abgeschieden, wodurch eine bessere Wechselwirkung mit dem Zielgas erreicht wird. Dadurch ergeben sich die schon zuvor beschriebenen Vorteile einer erhöhten Detektionsgeschwindigkeit und einer verbesserten Detektionssensitivität.

Bei einer weiteren Ausführungsform ist vorgesehen, dass zuerst der gasochrome Stoff an den SiO₂-Partikeln angeordnet wird. Darauffolgend werden die SiO₂-Partikel, welche vorzugsweise mit dem gasochromen Stoff bedeckt sind, an der Trägeroberfläche angeordnet. Diese Ausführungsform ermöglicht eine alternative Prozessführung, die besonders kostengünstig und effektiv ist.

Bei einer bevorzugten Ausführungsform wird der gasochrome Stoff auf der Trägerschicht aufgesintert und/oder aufgeschmolzen, vorzugsweise nach dem Abscheiden des gasochromen Stoffs auf den SiO₂-Partikeln.

Besonders bevorzugt erfolgt das Aufsintern und/oder das Aufschmelzen in einer Zeitspanne zwischen größer gleich 30 Sekunden und kleiner gleich 30 Minuten, höchst vorzugsweise für 2 Minuten. Dadurch wird der gasochrome Stoff nicht komplett in das Volumen eines SiO₂-Partikels aufgenommen, sondern verbleibt stattdessen an der äußeren Oberfläche der SiO₂-Partikel. Somit wird die zuvor genannte Zugänglichkeit des gasochromen Stoffs für das Zielgas nicht maßgeblich beeinträchtigt. Durch diese Ausführungsform ist der gasochrome Stoff vorteilhafterweise robuster gegenüber Abrieb, Erosion oder sonstigen Krafteinwirkungen, wodurch die Lebensdauer des gasochrom beschichteten Glases und des Gasdetektors vorteilhafterweise erhöht wird.

Erfindungsgemäß ist die Verwendung der zuvor genannten Vorrichtung, vorzugsweise eines Gasdetektors, zur Detektion eines Zielgases.

Es wurde außerdem erkannt, dass eine Anwendung in den Bereichen Smart Environment und/oder Umweltmesstechnik besonders bevorzugt ist.
Das gasochrom beschichtete Glas wird vorzugsweise als Fensterglas verwendet, insbesondere als Fensterglas an einem Gebäude oder an einem Fahrzeug. Das gasochrom beschichtete Glas und/oder die Vorrichtung zur Detektion eines Zielgases werden bevorzugt in einem tragbaren Kommunikationsgerät, insbesondere einem mobilen Telefon wie beispielsweise einem Smartphone und/oder an einer Hülle eines solchen Gerätes verwendet. Dadurch kann das Kommunikationsgerät vorteilhafterweise zur Detektion von Zielgas(en) eingesetzt werden, insbesondere zur Detektion von Umweltbelastungen (zum Beispiel mit Stickoxiden, NOₓ). Weitere Vorteile der Verwendungen ergeben sich durch die zuvor beschriebenen vorteilhaften Eigenschaften des gasochrom beschichteten Glases.

Weitere Eigenschaften und Vorteile der Erfindung ergeben sich aus der nachfolgenden Beschreibung anhand von Ausführungsbeispielen und anhand der Zeichnung.
- Figur 1a: zeigt ein erstes Ausführungsbeispiel eines erfindungsgemäßen gasochrom beschichteten Glases;
- Figur 1b: zeigt ein zweites Ausführungsbeispiel eines gasochrom beschichteten Glases mit Suprapartikeln;
- Figuren 2a, 2b: zeigen schematisch ein Ausführungsbeispiel eines erfindungsgemäßen Verfahrens zur Herstellung eines gasochromen Glases;
- Figuren 3a, b: zeigen schematisch ein weiteres Ausführungsbeispiel eines erfindungsgemäßen Verfahrens zur Herstellung eines gasochrom beschichteten Glases;
- Figur 4: zeigt schematisch ein weiteres Ausführungsbeispiel eines erfindungsgemäßen Verfahrens zur Herstellung eines gasochrom beschichteten Glases, bei dem ein Erhitzen erfolgt.
- Figur 5: zeigt ein Smartphone mit einem Ausführungsbeispiel einer erfindungsgemäßen Vorrichtung, welche als Gasdetektor ausgebildet ist;
- Figuren 6a, b: zeigen unterschiedliche Ausführungsformen des Gasdetektors der Figur 5;
- Figur 7: zeigt ein Ausführungsbeispiel einer erfindungsgemäßen Vorrichtung zur Detektion eines Zielgases, welche als Gasdetektor ausgebildet ist;
- Figur 8: zeigt ein weiteres Ausführungsbeispiel einer erfindungsgemäßen Vorrichtung zur Detektion eines Zielgases, welche als Gasdetektor ausgebildet ist, mit einer zielgasdurchlässigen Schutzschicht über der Trägerschicht; und
- Figur 9: zeigt ein weiteres Ausführungsbeispiel einer erfindungsgemäßen Vorrichtung zur Detektion eines Zielgases, welche als Gasdetektor ausgebildet ist, in Transmissionsmesskonfiguration.

In den Figuren verwendete gleiche Bezugszeichen bezeichnen gleiche oder zumindest gleichwirkende Elemente.

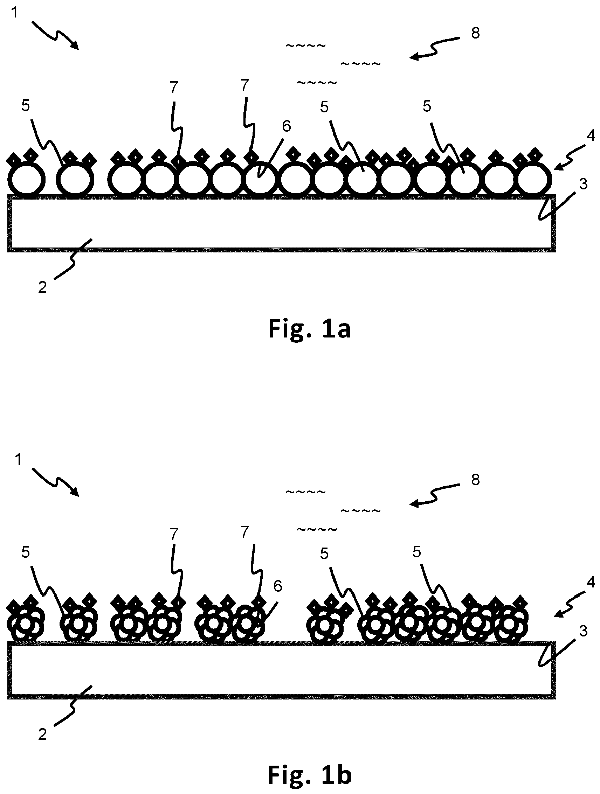

Figur 1a zeigt ein gasochrom beschichtetes Glas 1 mit einem Träger 2. Der Träger 2 weist eine Trägeroberfläche 3 auf. Die Trägeroberfläche 3 ist glatt, also nicht strukturiert oder profiliert. An der Trägeroberfläche 3 ist eine poröse Trägerschicht 4 aus SiO₂-Partikeln 5 angeordnet. Im Beispiel der Figur 1a ist die Trägerschicht 4 im rechten Bereich der Trägeroberfläche 3 aus einer deckenden Schicht von SiO₂-Partikeln 5 gebildet. Dabei berühren sich die einzelnen SiO₂-Partikel 5. Im linken Bereich der Trägeroberfläche 3 sind die SiO₂-Partikel 5 der Trägerschicht 4 etwas weiter voneinander getrennt, sodass sie sich gegenseitig nicht berühren. An einer äußeren porösen Oberfläche 6 der SiO₂-Partikel 5 ist ein gasochromer Stoff 7 angeordnet. Dabei ist die individuelle Beladung eines SiO₂-Partikels 5 mit gasochromen Stoff 7 verglichen zu einem anderen SiO₂-Partikel 5 unterschiedlich hoch. Der gasochrome Stoff 7 ist also über die SiO₂-Partikel 5 nicht gleich verteilt.

Bei einem alternativen Ausführungsbeispiel (nicht gezeigt) ist der gasochrome Stoff 7 an der porösen Oberfläche 6 der SiO₂-Partikel 5 gleich verteilt.

Im Beispiel der Figur 1a ist der gasochrome Stoff 7 zudem in Kontakt mit einem Zielgas 8, welches den gasochromen Stoff 7 umgibt. Insbesondere überströmt das Zielgas 8 das gasochrom beschichtete Glas 1. Durch die Anwesenheit des Zielgases 8 ändert der gasochrome Stoff 7 seine Farbe, wodurch die Anwesenheit des (sonst optisch nicht sichtbaren Zielgases 8) detektiert wird.

Figur 1b zeigt ein gasochrom beschichtetes Glas 1 bei dem die SiO₂-Partikel 5 als Suprapartikel 5 ausgebildet sind. Die Suprapartikel 5 sind Agglomerate aus SiO₂-Nanopartikeln. Wie aus der Figur 1b einfach ersichtlich wird, ist die äußere poröse Oberfläche 6 der Suprapartikel 5 im Vergleich zu der äußeren porösen Oberfläche 6 der SiO₂-Partikel 5 der Figur 1a vorteilhaft vergrößert. Dadurch wird eine höhere Beladung mit gasochromen Stoff 7 erreicht.

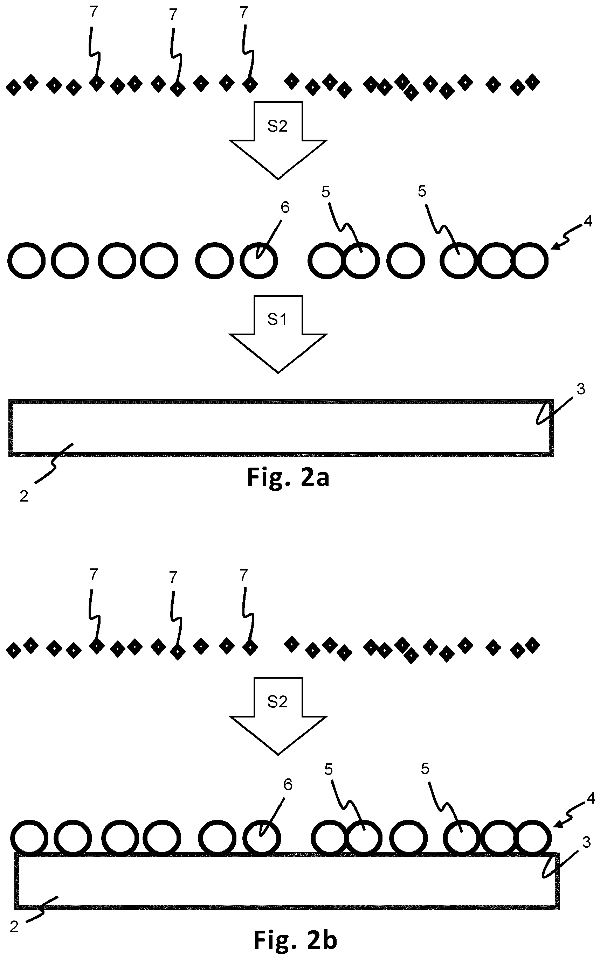

Die Figuren 2a und 2b zeigen schematisch ein Verfahren zur Herstellung eines gasochrom beschichteten Glases 1. Dabei wird gemäß Figur 2a in einem ersten Schritt S1 zunächst eine Vielzahl von SiO₂-Partikeln 5 an einer Trägeroberfläche 3 angeordnet und in einem zweiten Schritt S2 der gasochrome Stoff 7 an der porösen Oberfläche 6 der SiO₂-Partikel 5 angeordnet. Dies geht insbesondere aus der Figur 2b hervor.

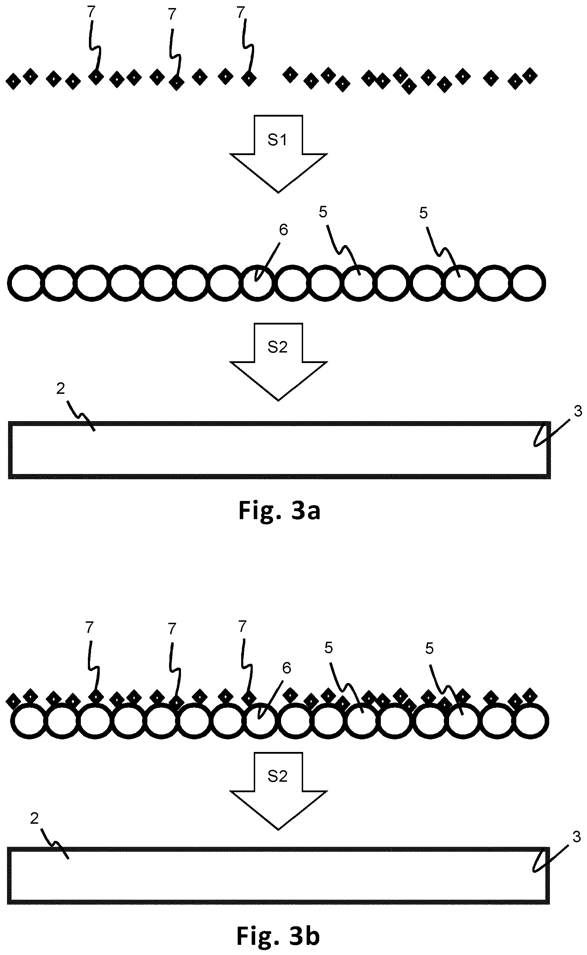

In einem alternativen Verfahren gemäß der Figuren 3a und 3b wird der gasochrome Stoff 7 in einem ersten Schritt S1 an der porösen Oberfläche 6 der SiO₂-Partikel 5 angeordnet (vergleiche Figur 3a). Darauffolgend werden in einem zweiten Schritt S2 die mit gasochromen Stoff 7 beschichteten SiO₂-Partikel 5 an der Trägeroberfläche 3 angeordnet.

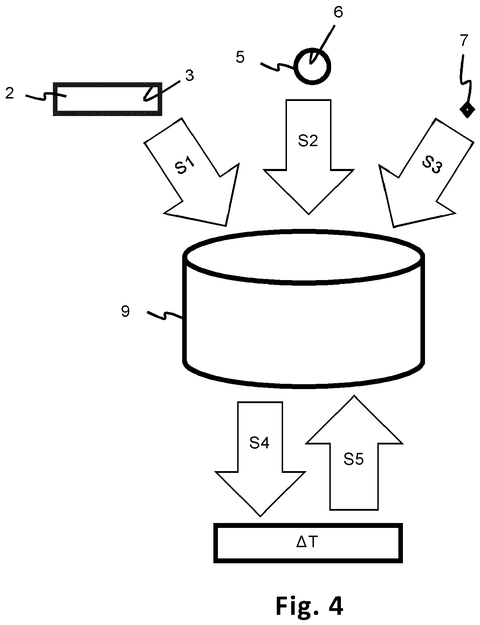

Figur 4 zeigt, dass die zuvor beschriebenen Verfahrensschritte insbesondere in einem Aufnahmegefäß 9 durchgeführt werden und vorzugsweise ein Erhitzen (ΔT) in einem Verfahrensschritt S4 erfolgt.

Dabei wird in einem ersten Ausführungsbeispiel im Verfahrensschritt S1 zuerst der Träger 2 in dem Aufnahmegefäß 9 vorgelegt. In einem darauffolgenden Verfahrensschritt S2 werden die SiO₂-Partikel 5 zugegeben. Die SiO₂-Partikel 5 werden dabei insbesondere in einem Lösungsmittel gelöst oder suspendiert. Die überstehende Lösung oder Suspension wird darauffolgend aus dem Aufnahmegefäß 9 abgelassen und/oder der Träger 2 wird aus dem Aufnahmegefäß 9 entnommen.

Nun erfolgt in einem darauffolgenden Schritt S4 ein Erhitzen, insbesondere ein Sintern bei einer Temperatur von zwischen 500 °C und 600 °C. Während des Erhitzens verbinden sich die SiO₂-Partikel 5 mit der Trägeroberfläche 3 stoffschlüssig. In einem nachfolgenden Schritt S5 wird der mit den SiO₂-Partikel 5 beschichtete Träger 2 wieder in das Aufnahmegefäß 9 gegeben. Nun wird der gasochrome Stoff 7 in einem Lösungsmittel gelöst oder suspendiert und in einem Schritt S3 in das Aufnahmegefäß 9 hinzugegeben. Darauffolgend wird die Lösung oder Suspension des gasochromen Stoffs 7 aus dem Aufnahmegefäß 9 abgelassen und/oder der Träger 2 aus dem Aufnahmegefäß 9 genommen. In einem nächsten Schritt erfolgt ein Trocknen, um noch etwaig vorhandene Lösungsmittelreste zu entfernen. Nach dem Trocknen oder statt dem Trocknen erfolgt optional auch ein Erhitzen gemäß S4, insbesondere ein Tempern bei 500 °C.

Bei thermisch instabilen gasochromen Stoffen 7 empfiehlt es sich, den Schritt S4 nicht durchzuführen. Auch in diesem Falle ist der gasochrome Stoff 7 durch die Abscheidung an der porösen Oberfläche 6 hinreichend gegenüber mechanischem Abrieb geschützt. Dennoch lässt sich durch ein Erhitzen (ΔT) gemäß S4 die Stabilität des gasochromen Stoffs 7 an der porösen Oberfläche 6 gegen mechanische Belastungen wie beispielsweise Abrieb weiter verbessern.

Bei einem weiteren Ausführungsbeispiel wird zuerst der Schritt S2 und dann der Schritt S3 durchgeführt, dies bedeutet, dass zunächst die SiO₂-Partikel 5 und der gasochrome Stoff 7 in dem Aufnahmegefäß 9 durchmischt werden und darauffolgend im Schritt S1 der Träger 2 in das Aufnahmegefäß 9 hinzugegeben wird. Nun wird der Träger 2 aus dem Aufnahmegefäß 9 entnommen und entweder dem zuvor beschriebenen Schritt des Trocknens oder dem Schritt des Erhitzens (ΔT) gemäß S4 unterworfen. Optional wird im Schritt S5 der bereits beschichtete Träger 2 nochmals in das Aufnahmegefäß 9 gegeben und gemäß Schritt S2 werden erneut SiO₂-Partikel 5 hinzugefügt, vorzugsweise in einem Lösungsmittel, das den gasochromen Stoffs 7 nicht abzulösen vermag. Im wiederholten Schritt S2 wird kein zusätzlicher gasochromer Stoff 7 beigefügt. Nach Entfernen des Trägers 2 aus dem Aufnahmegefäß 9 resultiert eine zweite Lage von SiO₂-Partikeln 5 über der zuvor angeordneten Trägerschicht 4. Ein derart modifiziert beschichtetes gasochromes Glas 1 weist insbesondere eine höhere Stabilität gegenüber Abrieb des gasochromen Stoffs 7 und/oder verbesserte Antireflexionseigenschaften auf.

Im Folgenden werden zwei Anwendungsbeispiele zur Herstellung eines gasochrom beschichteten Glases 1 beschrieben.

### Erstes Anwendungsbeispiel:

1. Sol-Gel Herstellung der SiO₂-Partikel 5:
   0,1 n Ammoniumhydroxidlösung in einer Menge von 1994 g werden mit 25670 g Ethanol vollständig vermischt und dazu unter weiterem Rühren 3405 g Tetramethoxysilan gegeben. Nach einer Rührzeit von 2 Stunden werden 26880 g wässriges, 2%iges Kieselsol zugegeben und 30 Minuten weitergerührt, bis noch 86925 g I-Methoxy-2-propanol dem Ansatz hinzugefügt werden. Das wässrige, 2%ige Kieselsol wird gemäß US Patent 4.775.520 hergestellt. Die Beschichtungslösung wird über Nacht gerührt und abschließend filtriert.
2. Anordnen der SiO₂-Partikel 5 an der Trägeroberfläche 3:
   In die Beschichtungslösung wird eine zuvor gereinigte Glasscheibe (Träger 2) getaucht und mit einer Geschwindigkeit von 15 cm/min herausgezogen.
3. Trocknen und nachfolgendes Erhitzen, insbesondere Tempern:
   Nach 10 min Ablüften wird die Glasscheibe (Träger 2) bei 550 °C für 15 min getempert. Es resultiert ein porös beschichtetes transparentes Glas (Träger 2 mit Trägerschicht 4).
4. Anordnen des gasochromen Stoffs 7 auf der porösen Oberfläche 6
   Ein zweikerniger Rhodiumkomplex (CO-Gassensorfarbstoff, gasochromer Stoff 7) wird in Toluol (Lösungsmittel) mit einer Konzentration von 0,3 Gew.-% gelöst. Die poröse Glasscheibe (Träger 2 mit Trägerschicht 4) wurde in die Lösung über Nacht getaucht. Die poröse Schicht (Trägerschicht 4) zeigte anschließend eine leicht violette Farbe. Der Überstand (der Lösung) wurde abgenommen.
5. Trocknen:
   Die resultierende, farbstoffmodifizierte Beschichtung (Trägerschicht 4 mit gasochromen Stoff 7) wird in einem Vakuumofen bei 60 °C und einem Druck von 10 mbar für 48 h getrocknet. Nach dem Trocknen zeigen die CO-Indikatoren (gasochrome Stoff 7) auf der porösen Schicht (Trägerschicht 4) einen rosa bis violetten Schimmer.

### Zweites Anwendungsbeispiel:

1. Sol-Gel Herstellung der SiO₂-Partikel 5:
   7,6 g Polyethylenglycol mit einer mittleren Molmasse von 10 000 werden in 9,5 g ammoniakalischem Wasser mit einem pH-Wert von 9,5 in Gegenwart von 27,0 g Methanol gelöst. Diese Lösung wird zu einem Gemisch aus 15,2 g Tetramethoxysilan und 80,0 g Methanol gegeben. Nach 10-minütigem Rühren wird die resultierende Mischung filtriert.
2. Anordnen der SiO₂-Partikel 5 an der Trägeroberfläche 3:
   Nach einer Alterungszeit von ca. 80 min werden Glasscheiben (Träger 2) durch Tauchen beschichtet. Zur Erzielung einer ganz besonders gleichmäßigen Schicht mit einer Schichtdicke zwischen größer gleich 50 nm und kleiner gleich 200 nm, vorzugsweise 100 nm, wird die zu beschichtende Scheibe im Beschichtungsbad (Aufnahmegefäß 9) fixiert und die darin befindliche Beschichtungslösung innerhalb von 2 min erschütterungsfrei abgeführt.
3. Trocknen und nachfolgendes Erhitzen, insbesondere Tempern:
   Nach dem Beschichtungsvorgang werden die Scheiben (Träger 2 mit Trägerschicht 4) 30 min bei 130 °C getrocknet, anschließend mit einer Heizrate von 120°K/h auf 500 °C gebracht und eine Stunde bei dieser Temperatur gehalten.
4. Anordnen des gasochromen Stoffs 7 auf der poröse Oberfläche 6:
   Ein zweikerniger Rhodiumkomplex (CO-Gassensorfarbstoff, gasochromer Stoff 7) wurde in Toluol (Lösungsmittel) mit einer Konzentration von 0,3 Gew.-% gelöst. Die poröse Glasscheibe (Träger 2 mit Trägerschicht 4) wurde in die Lösung über Nacht getaucht. Die poröse Schicht (Trägerschicht 4) zeigte anschließend eine leicht violette Farbe. Der Überstand (der Lösung) wurde abgenommen.
5. Trocknen:
   Die resultierende, farbstoffmodifizierte Beschichtung (Trägerschicht 4 mit gasochromen Stoff 7) wurde in einem Vakuumofen bei 60 °C und einem Druck von 10 mbar für 48 h getrocknet. Nach dem Trocknen zeigten die CO-Indikatoren (gasochrome Stoff 7) auf der porösen Schicht (Trägerschicht 4) einen rosa bis violetten Schimmer.

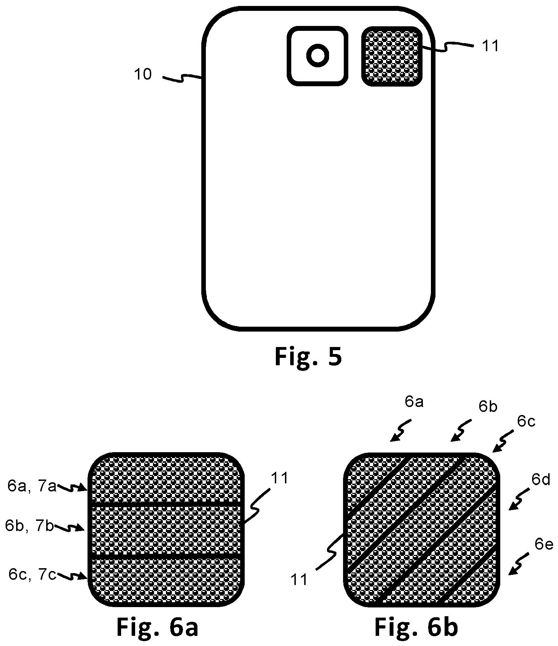

Figur 5 zeigt ein Smartphone 10 mit einem Gasdetektor 11. Der Gasdetektor 11 umfasst ein gasochrom beschichtetes Glas 1 (vgl. Figuren 1a oder 1b). Dabei ist der Gasdetektor 11 entweder in das Smartphone 10 integriert oder beispielsweise an einer externen Hülle des Smartphones 10 angeordnet. Das Anwendungspotenzial des Smartphones 10 mit Gasdetektor 11 erstreckt sich insbesondere auf die Bereiche Smart Environment und/oder Umweltmesstechnik, wobei mit dem Smartphone 10 beispielsweise aktuelle Umweltbelastungen (zum Beispiel mit Stickoxiden, NOₓ) erfasst und/oder gemessen werden können.

Bei einem weiteren, nicht gezeigten, Ausführungsbeispiel wird ein gasochrom beschichtetes Glas 1 als Fensterglas verwendet. Durch die Farbänderung eines gasochrom beschichteten Fensterglases werden insbesondere Hinweise über die aktuelle Umweltbelastung (z. B. NO₂) erhalten. Ein gasochrom beschichtetes Fensterglas dient insbesondere als Messpunkt in einer Immissionskarte.

Die Figur 6a zeigt ein Ausführungsbeispiel eines Gasdetektors 11 aus der Figur 5. Dabei weist die poröse Oberfläche 6 (vgl. Figuren 1a oder 1b) des gasochrom beschichteten Glases 1 (vgl. Figuren 1a oder 1b) einen ersten Teilbereich 6a, einen zweiten Teilbereich 6b und einen dritten Teilbereich 6c auf. Im ersten Teilbereich 6a ist ein erster gasochromer Stoff 7a angeordnet, wohingegen im zweiten Teilbereich 6b und im dritten Teilbereich 6c jeweils unterschiedliche gasochrome Stoffe 7b beziehungsweise 7c angeordnet sind. Mittels der unterschiedlichen gasochromen Stoffe 7a-c lassen sich unterschiedliche Zielgase 8 (vgl. Figur 1a) detektieren. Somit wird der Gasdetektor 11 der Figur 6a zur Detektion von drei unterschiedlichen Zielgasen 8 eingesetzt, beispielsweise reagiert der erste gasochrome Stoff 7a im ersten Teilbereich 6a mit NO₂, der zweite gasochrome Stoff 7b im zweiten Teilbereich 6b mit CO und der dritte gasochrome Stoff 7c im dritten Teilbereich 6c mit SO₂.

In der Figur 6b ist eine alternative Ausführungsform eines Gasdetektors 11 gezeigt, bei dem neben dem ersten Teilbereich 6a, dem zweiten Teilbereich 6b und dem dritten Teilbereich 6c auch noch weitere Teilbereiche 6d und 6e der porösen Oberfläche 6 umfasst sind. Dabei ist im ersten Teilbereich 6a ein gasochromer Stoff 7 (vgl. Figur 1a) in einer ersten Konzentration angeordnet und in dem zweiten Teilbereich 6b der gleiche gasochrome Stoff 7 in einer zweiten, insbesondere höheren, Konzentration angeordnet. Auch in den übrigen Teilbereichen 6c bis 6e ist der gasochrome Stoff 7 in unterschiedlichen Konzentrationen an der porösen Oberfläche 6 angeordnet. Dabei steigt die Konzentration des gasochromen Stoffs 7 in der Reihenfolge der Teilbereiche von 6a bis 6e graduell an (Konzentrationsgradient).

Durch einen derartigen Gasdetektor 11 lässt sich insbesondere eine konzentrationsabhängige Detektion des Zielgases 8 (vgl. Figur 1a) erreichen. Bei einer niedrigen Konzentration des Zielgases 8 ist die Farbänderung und/oder der Farbumschlag bevorzugt im Teilbereich 6e detektierbar, da Teilbereich 6e die höchste Konzentration des gasochromen Stoffes 7 (vgl. Figur 1a) aufweist. Dies ist dadurch zu begründen, dass das Zielgas 8 den Gasdetektor 11 gleichmäßig umströmt und mit dem gasochromen Stoff 7 der porösen Oberfläche 6 reagiert. Die Reaktionswahrscheinlichkeit ist für alle Teilbereiche 6a bis e gleich groß, sodass bei einer niedrigen Zielgaskonzentration eine intensiv messbare Detektion vorzugsweise in dem Teilbereich 6e erfolgt. Bei einer höheren Konzentration des Zielgases 8 lässt sich die Farbänderung auch in den anderen Teilbereichen 6d bis 6a (in dieser Reihenfolge) detektieren. Somit kann nach einer Kalibration des Gasdetektors 11 der Figur 6b mit vorbekannten Zielgaskonzentrationen eine quantitative Messung/Bestimmung von unbekannten Zielgaskonzentrationen erfolgen.

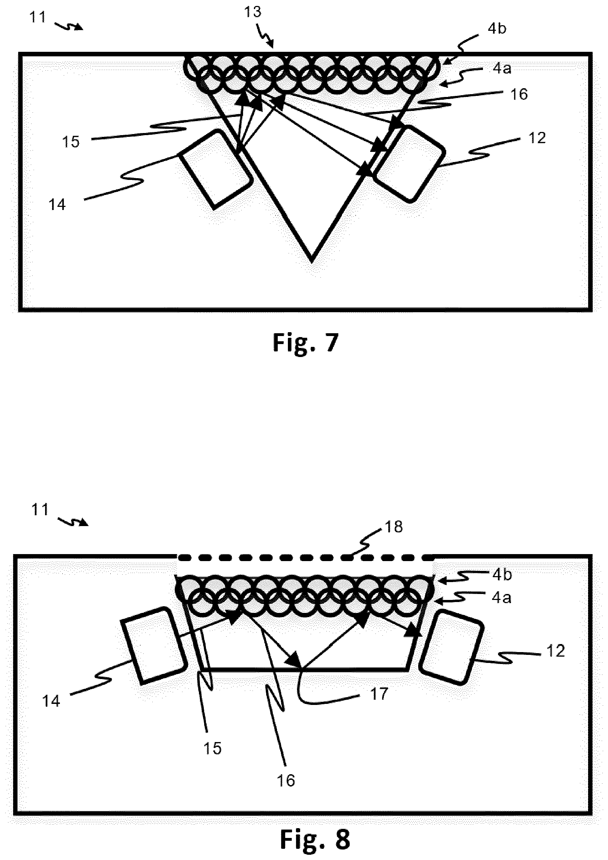

Figur 7 zeigt eine Vorrichtung zur Detektion eines Zielgases 8, welche einem Gasdetektor 11 entspricht. Der Gasdetektor 11 umfasst ein gasochrom beschichtetes Glas 1 (vgl. Figuren 1a, 1b) mit einer ersten Lage 4a von SiO₂-Partikeln 5 und zumindest einer zweiten Lage 4b von SiO₂-Partikeln 5. Der Gasdetektor 11 umfasst zudem einen Detektor 12, welcher in Messkonfiguration des gasochromen Stoffs 7 (vgl. Figur 1a) in einem Detektionsbereich 13, angeordnet ist. Der Gasdetektor 11 weist zudem eine interne Lichtquelle 14 auf, welche einen Lichtstrahl 15 auf den Detektionsbereich 13 emittiert. Der Lichtstrahl 15 durchdringt dabei den Träger 2 des gasochrom beschichteten Glases 1 (vgl. Figur 1a) und eine erste Lage 4a aus SiO₂-Partikeln 5 (vgl. Figur 1a), an denen der gasochrome Stoff 7 angeordnet ist. Durch eine Wechselwirkung des Lichtstrahls 15 mit dem gasochromen Stoff 7, beispielsweise durch Absorption, ändert der Lichtstrahl 15 seine Intensität, zumindest in einem bestimmten Wellenlängenbereich. Der Lichtstrahl 15 trifft darauffolgend auf eine zweite Lage 4b, welche über der ersten Lage 4a angeordnet ist. Dabei wird der Lichtstrahl 15 an der zweiten Lage 4b reflektiert. Der reflektierte Lichtstrahl 16 fällt auf den Detektor 12 ein, welcher die Intensitätsänderung des reflektierten Lichtstrahls 16 (nach der Wechselwirkung mit dem gasochromen Stoff 7) verglichen zu dem emittierten Lichtstrahl 15, detektiert.

Figur 8 zeigt einen alternativen Gasdetektor 11, bei dem der emittierte Lichtstrahl 15 an der Trägerschicht 4 mit dem gasochromen Stoff 7, wie zuvor beschrieben, wechselwirkt und darauffolgend reflektiert wird. Der reflektierte Lichtstrahl 16 wird an einer verspiegelten Innenfläche 17 erneut reflektiert, trifft erneut auf die Trägerschicht 4 und nach erneuter Reflektion auf den Detektor 12 ein, welcher die Intensitätsänderung detektiert.

Der Gasdetektor 11 der Figur 8 weist zudem eine Schutzschicht 18 auf, welche die Trägerschicht 4 vor Beschädigungen schützt, insbesondre vor Abrieb. Die Schutzschicht 18 ist zielgasdurchlässig, wodurch das Zielgas 8 an den gasochromen Stoff 7 gelangt und auch wieder davon weg diffundiert.

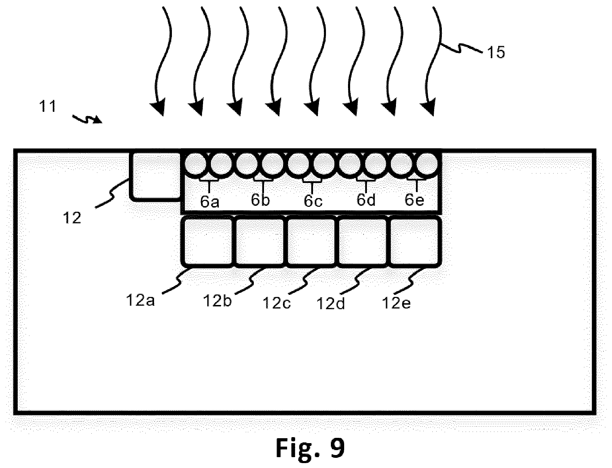

Figur 9 zeigt einen Gasdetektor 11 bei dem extern einfallende Lichtstrahlen 15 genutzt werden. Der Gasdetektor 11 umfasst einen Detektor 12 und weitere Detektoren 12a bis e. Dabei ist der Detektor 12 in Messkonfiguration zur Bestimmung einer Intensität des einfallenden Lichtstrahls 15 angeordnet. Im Beispiel der Figur 9 entspricht der einfallende Lichtstrahl 15 dem Sonnenlicht. Die Detektoren 12a bis e sind in Transmissionsmesskonfiguration angeordnet. Dabei ist jeder einzelne der Detektoren 12a bis 12e einem Teilbereich 6a bis 6e der porösen Oberfläche 6 (vgl. Figur 1a) zugeordnet. Durch eine derartige Anordnung werden, wie bereits bei den Figuren 6a und b beschrieben, zum einen unterschiedliche Zielgase 8 detektiert und zum anderen auch die Konzentration(en) eines Zielgases 8 und/oder von mehreren Zielgasen 8 bestimmt.

### Bezugszeichen

- 1: gasochrom beschichtetes Glas
- 2: Träger
- 3: Trägeroberfläche
- 4: Trägerschicht
- 4a: erste Lage
- 4b: zweite Lage
- 5: SiO₂-Partikel
- 6: porösen Oberfläche
- 6a: erster Teilbereich der porösen Oberfläche
- 6b: zweiter Teilbereich der porösen Oberfläche
- 6c: dritter Teilbereich der porösen Oberfläche
- 6d-e: weitere Teilbereiche der porösen Oberfläche
- 7: gasochromer Stoff
- 7a: erster gasochromer Stoff
- 7b: zweiter gasochromer Stoff
- 7c: dritter gasochromer Stoff
- 8: Zielgas
- 9: Aufnahmegefäß
- 10: Smartphone
- 11: Gasdetektor
- 12: Detektor
- 12a-e: weitere Detektoren
- 13: Detektionsbereich
- 14: Lichtquelle
- 15: Lichtstrahl
- 16: reflektierter Lichtstrahl
- 17: verspiegelte Innenfläche
- 18: Schutzschicht

## Patentansprüche

1. Gasochrom beschichtetes Glas (1) für einen Gasdetektor (11),
mit einem Träger (2) mit einer Trägeroberfläche (3); und
einem gasochromen Stoff (7);
**dadurch gekennzeichnet,**
**dass** eine poröse Trägerschicht (4), welche SiO₂-Partikel (5) aufweist, an der Trägeroberfläche (3) angeordnet ist und
**dass** der gasochrome Stoff (7) an den SiO₂-Partikeln (5) der porösen Trägerschicht (4) angeordnet ist.

2. Gasochrom beschichtetes Glas (1) nach Anspruch 1,
wobei der gasochrome Stoff (7) nicht in ein Polymer eingebettet ist.

3. Gasochrom beschichtetes Glas (1) nach Anspruch 1 oder 2, welches frei von organischen Polymeren ist.

4. Gasochrom beschichtetes Glas (1) nach einem der Ansprüche 1 bis 3, bei dem der gasochrome Stoff (7) dazu ausgebildet ist, durch die Einwirkung eines Zielgases (8) eine geänderte Farbe aufzuweisen und
die poröse Trägerschicht (4) transparent für Licht zumindest im Wellenlängenbereich der geänderten Farbe ist.

5. Gasochrom beschichtetes Glas (1) nach einem der Ansprüche 1 bis 4, bei dem die poröse Trägerschicht (4) mit der Trägeroberfläche (3) stoffschlüssig verbunden ist.

6. Gasochrom beschichtetes Glas (1) nach einem der Ansprüche 1 bis 5, bei dem die SiO₂-Partikel (5) als Agglomerate von SiO₂-Nanopartikeln ausgebildet sind, vorzugsweise als Suprapartikel.

7. Gasochrom beschichtetes Glas (1) nach einem der Ansprüche 1 bis 6, bei dem die poröse Trägerschicht (4) zumindest einen ersten Teilbereich (6a) und einen zweiten Teilbereich (6b) umfasst; und
der gasochrome Stoff (7) einen ersten gasochromen Stoff (7a) und einen zweiten gasochromen Stoff (7b) umfasst; wobei
der erste Teilbereich (6a) und der zweite Teilbereich (6b) räumlich voneinander getrennt sind, und
der erste gasochrome Stoff (7a) an dem ersten Teilbereich (6a) und der zweite gasochrome Stoff (7b) an dem zweiten Teilbereich (6b) angeordnet ist.

8. Gasochrom beschichtetes Glas (1) nach einem der Ansprüche 1 bis 7, bei dem die SiO₂-Partikel (5) als Suprapartikel ausgebildet sind,
welche einen Durchmesser von größer gleich 4 µm bis kleiner gleich 100 µm aufweisen, vorzugsweise größer gleich 5 µm bis kleiner gleich 80 µm, besonders vorzugsweise größer gleich 30 µm bis kleiner gleich 70 µm; und/oder
eine spezifische Oberfläche von größer gleich 40 m²g⁻¹ bis kleiner gleich 180 m²g⁻¹ aufweisen, vorzugsweise größer gleich 45 m²g⁻¹ bis kleiner gleich 100 m²g⁻¹.

9. Gasochrom beschichtetes Glas (1) nach einem der Ansprüche 1 bis 8, bei dem die poröse Trägerschicht (4) zumindest eine erste Lage (4a) von SiO₂-Partikeln (5) und zumindest eine zweite Lage (4b) von SiO₂-Partikeln (5) umfasst; wobei die erste Lage (4a) zwischen dem Träger (2) und der zweiten Lage (4b) angeordnet ist; und
der gasochrome Stoff (7) an den SiO₂-Partikeln (5) der ersten Lage (4a) angeordnet ist.

10. Vorrichtung zur Detektion eines Zielgases (8), vorzugsweise ein Gasdetektor (11), umfassend
ein gasochrom beschichtetes Glas (1) nach einem der Ansprüche 1 bis 9; einen Detektor (12); und
einen Detektionsbereich (13);
wobei der gasochrome Stoff (7) zumindest in dem Detektionsbereich (13) angeordnet ist; und
der Detektor (12) dazu ausgebildet ist, abhängig von einer Farbänderung des Detektionsbereichs (13) das Zielgas (8) zu detektieren.

11. Verfahren zur Herstellung eines gasochrom beschichteten Glases (1), vorzugsweise nach einem der Ansprüche 1 bis 9, umfassend die Schritte:
- Bereitstellen eines Trägers (2) mit einer Trägeroberfläche (3);
einer Vielzahl von SiO₂-Partikeln (5); und
eines gasochromen Stoffs (7);
- Anordnen der SiO₂-Partikel (5) an der Trägeroberfläche (3);
- Anordnen des gasochromen Stoffs (7) an den SiO₂-Partikeln (5).

12. Verfahren zur Herstellung eines gasochrom beschichteten Glases (1) nach Anspruch 11, bei dem
kein Einbetten des gasochromen Stoffs (7) in ein Polymer erfolgt.

13. Verfahren zur Herstellung eines gasochrom beschichteten Glases (1) nach Anspruch 11 oder 12, bei dem nach dem Anordnen der SiO₂-Partikel (5) an der Trägeroberfläche (3) ein
- Erhitzen erfolgt, vorzugsweise ein Tempern bei einer Temperatur in einem Bereich von größer gleich 300 °C bis kleiner gleich 700 °C, besonders vorzugsweise bei 500 °C; und
bevorzugt der gasochrome Stoff (7) nachfolgend an den SiO₂-Partikeln (5) angeordnet wird.

14. Verwendung einer Vorrichtung nach Anspruch 10 zur Detektion eines Zielgases (8).
